# EUROPEAN PATENT APPLICATION

(11) **EP 4 484 423 A1**
(43) Date of publication of application: **01.01.2025**
(21) Application number: 23759166.4
(22) Date of filing: 21.02.2023
(51) Int. Cl.: C07D 413/12, C07D 267/10

(54) **PREPARATION METHOD OF NITROGEN-CONTAINING HETEROCYCLIC COMPOUND**

(30) Priority: 22.02.2022 CN 202210160847
(71) Applicant: Haisco Pharmaceuticals Pte. Ltd., Singapore 079903 (SG)
(72) Inventor: FAN, Jiang, Chengdu, Sichuan 611130 (CN); DOU, Ying, Chengdu, Sichuan 611130 (CN); ZHU, Fengfei, Chengdu, Sichuan 611130 (CN); WANG, Chengtao, Chengdu, Sichuan 611130 (CN); GAN, Man, Chengdu, Sichuan 611130 (CN)
(74) Representative: IP TRUST SERVICES
(86) International application number: PCT/CN2023/077408
(87) International publication number: WO 2023/160541

(57) **Abstract**

Disclosed is a preparation method of a nitrogen-containing heterocyclic compound represented by formula (I). The method comprises: carrying out a coupling reaction on a compound 1A and a compound 2a, and sequentially carrying out deprotection, amidation and deprotection reactions, which are four steps in total, to obtain a target compound. The method is short in reaction route, mild in condition, simple to operate, convenient in post-treatment, high in yield and high in purity, and is suitable for industrial amplification production.

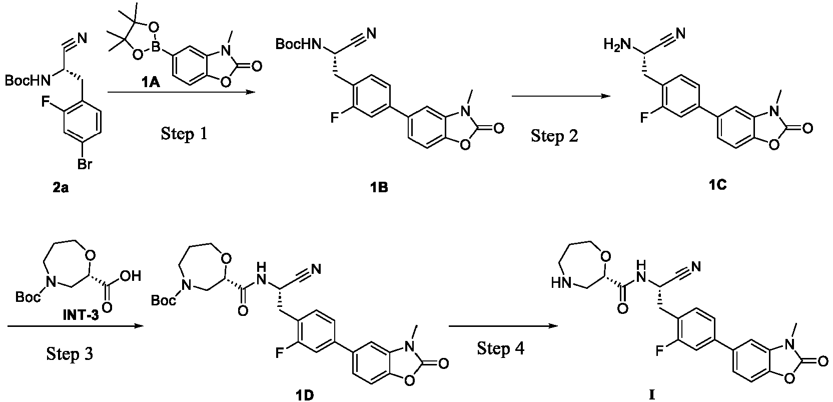

## Description

### Technical Field

The present invention relates to a preparation method of a nitrogen-containing heterocyclic compound.

### Background Art

Dipeptidyl peptidase 1 (DPP1), also known as cathepsin C, is a cysteinyl protease of the lysosomal papain family involved in intracellular protein degradation. During neutrophil maturation, DPP1 activates neutrophil serine proteases (NSPs), including neutrophil elastase (NE), proteinase 3 (Pr3), and cathepsin G (CatG), by cleaving the N-terminal dipeptide of a target protein. DPP1 has been implicated in a variety of inflammatory diseases, including Wegener's granulomatosis, rheumatoid arthritis, lung inflammation and virus infection. Studies have shown that the inhibition of DPP1 can have a good therapeutic effect on highly inflammatory lung diseases caused by neutrophils, such as bronchiectasis, chronic obstructive pulmonary disease (COPD) and acute lung injury. Therefore, inhibiting the over-activation of NSPs by targeting DPP1 may have a potential therapeutic effect on bronchiectasis.

In patent application PCT/CN2020/114500, a DPP1 small molecule inhibitor of formula (I) is prepared. The compound exhibits relatively high DPP1 inhibitory activity and excellent bioavailability and pharmacokinetic characteristics and has the advantages of low toxicity and high safety. It is intended to be used to treat lung diseases such as non-cystic fibrosis bronchiectasis, chronic obstructive pulmonary disease (COPD), acute lung injury and cystic fibrosis bronchiectasis. However, in the preparation method described in the patent, the purification of the intermediates is difficult and requires multiple steps of column purification, many reaction conditions are unfavourable for large-scale production, and there are also disadvantages such as low yield and high cost. Therefore, it is necessary to improve the preparation process to facilitate large-scale industrial production.

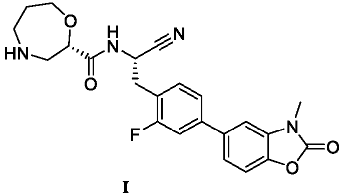

### Summary of the Invention

The present invention provides a preparation method of a compound of formula (I), 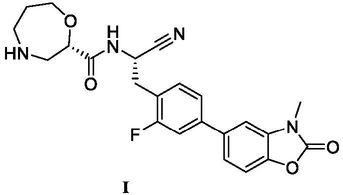
comprising: step 1: subjecting compound 2a and compound 1A to a coupling reaction to obtain intermediate 1B; 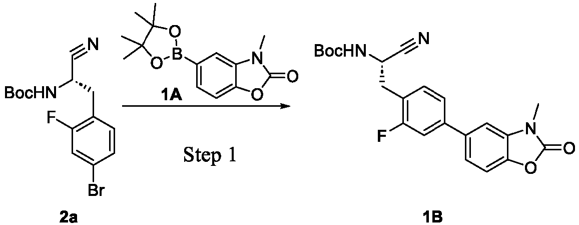
and step 2: subjecting the intermediate 1B to deprotection to obtain intermediate 1C or a salt thereof;

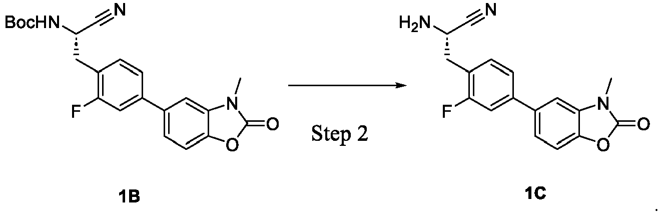

Starting compounds 2a and 1A can be synthesized with reference to the methods described in existing chemical reference books, literature, patents, etc., or can be purchased commercially. For example, the preparation method of compound 1A is described in patent WO 2015110826 A1 (page 50), and the preparation method of compound 2a is described in WO 2016139355 A1 (page 52).

The coupling reaction in step 1 can generally be performed under alkaline conditions using a catalyst such as palladium or nickel. The palladium or nickel reagent includes, but is not limited to one or more of NiCl₂, NiCl₂·diglyme (diglycol dimethyl ether nickel dichloride), Ni(COD)₂, Pd(OAc)₂, PdCl₂, Pd(PPh₃)₂Cl₂, Pd(dppf)Cl₂, Pd(PPh₃)₄, Pd₂(dba)₃, Pd(PhCN)₂Cl₂, PEPPSI-iPr, PdCl₂[P(Cy)₃]₂ or [1,1'-bis(diphenylphosphino)ferrocene]palladium dichloride dichloromethane complex (Pd(dppf)Cl₂·CH₂Cl₂); alkaline reagents for creating alkaline conditions include, but are not limited to, K₃PO₄, K₂HPO₄, K₂CO₃, KHCO₃, Cs₂CO₃, Na₂CO₃, NaHCO₃, KF, sodium acetate, potassium acetate, pyridine, triethylamine or N,N-diisopropylethylamine, or an aqueous solution thereof;
in some specific embodiments, the step 1 comprises dissolving compound 2a and compound 1A in an organic solvent, wherein the organic solvent is an organic solvent that is capable of dissolving the reactants, and includes, but is not limited to one or more of acetonitrile, toluene, dichloromethane, ethyl acetate, acetone, methanol, ethanol, isopropanol, 2-methyl tetrahydrofuran, tetrahydrofuran, and 1,4-dioxane; then adding one or more of K₃PO₄, K₂HPO₄, K₂CO₃, KHCO₃, Cs₂CO₃, Na₂CO₃, NaHCO₃, KF, sodium acetate, potassium acetate, pyridine, triethylamine or N,N-diisopropylethylamine, or an aqueous solution thereof; after the addition, adding one or more catalysts selected from NiCl₂, NiCl₂·diglyme, Ni(COD)₂, Pd(OAc)₂, PdCl₂, Pd(PPh₃)₂Cl₂, Pd(dppf)Cl₂, Pd(PPh₃)₄, Pd₂(dba)₃, Pd(PhCN)₂Cl₂, PEPPSI-iPr, PdCl₂[P(Cy)₃]₂, and [1,1'-bis(diphenylphosphino)ferrocene]palladium dichloride dichloromethane complex (Pd(dppf)Cl₂CH₂Ch); under nitrogen protection, warming the mixture to 80°C ± 5°C for a complete reaction; then, adding purified water or ethanol to the reaction liquid, or directly cooling the mixture to 5°C-55°C, and performing filtration and drying to obtain intermediate 1B; optionally, after filtration, adding the filter cake to an alcohol solvent, stirring the mixture for 10 min to 2 h, and then performing filtration and drying to obtain the intermediate 1B. Unless otherwise specified, the alcohol solvent includes, but is not limited to methanol, ethanol, propanol, isopropanol, n-butanol, etc. Under the reaction conditions, the reaction in the step has a fast rate, high conversion, simple post-treatment and high product purity.

In some specific embodiments, in step 1, the reaction system is subjected to nitrogen replacement prior to the addition of a catalyst, and after the addition, another nitrogen replacement is performed with the aim of substantially removing oxygen from the reaction system and avoiding catalyst deactivation.

In some specific embodiments, the feeding ratio range of the reaction in step 1 is: 2a : 1A : palladium reagent = 1 : 1.0-3.0 : 0.001-0.2, preferably 2a : 1A : palladium reagent = 1 : 0.95-1.20 : 0.001-0.03, and potassium carbonate is added in the following amount range: 2a : K₂CO₃ = 1.0 : 1.5-8.0, preferably 2a : K₂CO₃ = 1.0 : 2.0-3.0.

In some specific embodiments, step 2 comprises: adding the intermediate 1B and an acid to an organic solvent, fully reacting the mixture while controlling the temperature at 20°C-55°C; then filtering same to obtain intermediate 1C or a salt thereof; optionally, adding the filter cake to an organic solvent, warming the mixture to 80°C ± 5°C and stirring same for 1 to 5 hours, cooling same to 20°C ± 5°C, stirring same and performing filtration and drying to obtain the intermediate 1C or the salt thereof, wherein the acid is selected from one or more of sulphuric acid, phosphoric acid, formic acid, trifluoroacetic acid, benzenesulphonic acid, methanesulphonic acid, and p-toluenesulphonic acid monohydrate, and the organic solvent is selected from one or more of methanol, ethanol, isopropanol, acetonitrile, isopropyl acetate, ethyl acetate, toluene, acetone, methyl isobutyl ketone, tetrahydrofuran, 2-methyl tetrahydrofuran, methyl tert-butyl ether and acetonitrile; and
the Boc deprotection reaction is generally performed at a controlled temperature after dissolving the intermediate and the acid in an organic solvent, wherein the acid includes, but is not limited to sulphuric acid, phosphoric acid, formic acid, trifluoroacetic acid, benzenesulphonic acid, methanesulphonic acid, p-toluenesulphonic acid monohydrate, etc., the organic solvent includes, but is not limited to methanol, ethanol, isopropanol, acetonitrile, isopropyl acetate, ethyl acetate, toluene, acetone, methyl isobutyl ketone, tetrahydrofuran, 2-methyl tetrahydrofuran, methyl tert-butyl ether, acetonitrile, etc., and the temperature is about 0°C-80°C.

In some specific embodiments of the present invention, step 2 comprises adding the intermediate 1B and p-toluenesulphonic acid monohydrate to acetonitrile, and fully reacting the mixture while controlling the temperature at 25°C ± 5°C; then filtering same, adding the filter cake to acetonitrile, warming the mixture to 80°C ± 5°C and stirring same for 1 to 5 hours, cooling same to 20°C ± 5°C, stirring same, and performing filtration and drying to obtain intermediate 1C or a salt thereof.

The reaction in step 2 of the present invention is preferably performed in acetonitrile using p-toluenesulphonic acid monohydrate as the acid. The advantages thereof lie in that the reaction has a fast rate, the product is directly precipitated at the end of the reaction and can be obtained by filtration, and the reaction is simple to operate. The post-treatment involves further stirring the product in acetonitrile at a high temperature, which has the advantages of excellent impurity removal after slurrying while heating and non-hygroscopic product.

In some specific embodiments, the feeding molar ratio of the reaction in step 2 is: 1B : acid = 1 : 1.0-5.0; and in some embodiments, the ratio is preferably 1B : acid = 1 : 1.5-3.0.

In some specific embodiments, the preparation method of formula (I) of the present invention further comprises
step 3: subjecting the intermediate 1C or the salt thereof and compound INT-3 or a salt thereof to an amidation reaction to obtain intermediate 1D; 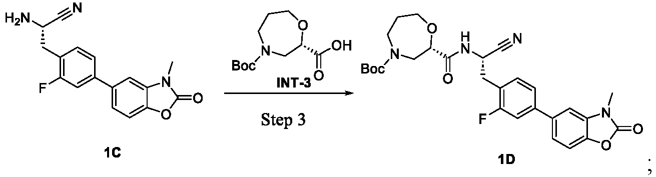 or/and
step 4: subjecting the intermediate 1D to deprotection under acidic conditions to obtain compound (I) or a salt thereof;

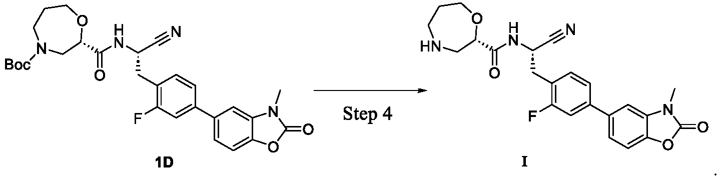

The amidation reaction in step 3 is generally a dehydration reaction performed in a condensation reagent under alkaline conditions.

In some specific embodiments, the step 3 comprises: adding the intermediate 1C or the salt thereof and the compound INT-3 or the salt thereof to an organic solvent, adding an organic amine, then adding an acid-amine condensation reagent under nitrogen protection while controlling the temperature at 5°C-20°C, and then fully reacting the mixture while maintaining the temperature at 25°C ± 5°C; then, washing the reaction liquid with sodium chloride solution, performing liquid separation, adding medicinal charcoal or activated carbon to the organic phase, stirring and filtering the mixture, drying the organic phase by adding anhydrous sodium sulphate, and performing filtration and concentration to obtain the intermediate 1D,
wherein the organic solvent is selected from one or more of tetrahydrofuran, 2-methyl tetrahydrofuran, dioxane, N,N-dimethylformamide, N,N-dimethylacetamide, N-methylpyrrolidone, dimethylsulphoxide, dichloromethane, acetone, methyl isobutyl ketone, isopropyl acetate and ethyl acetate;
the organic amine is selected from one or more of triethylamine, 2,6-dimethylpyridine, pyridine, DBU and N,N-diisopropylethylamine;
and the acid-amine condensation reagent is selected from one or more of HATU, COMU, EDCI, BOP and propylphosphonic anhydride.

In some specific embodiments, the material ratio range in the step 3 is: 1C : INT-3 = 1 : 0.9-3.0, preferably 1C : INT-3 = 1 : 1.0 to 1.5, and the condensation reagent is generally added in the following amount range: 1C : condensation reagent = 1 : 1.0-5.0, preferably 1C : condensation reagent = 1 : 1.2-2.0.

In some specific embodiments, the step 3 comprises: sequentially washing the reaction liquid with an alkaline aqueous solution and an acidic aqueous solution before washing same with sodium chloride solution, wherein the alkaline aqueous solution is selected from one or more of potassium phosphate solution, potassium carbonate solution, potassium bicarbonate solution, sodium carbonate solution and sodium bicarbonate solution. The washing step is intended to remove unreacted 1C or INT-3 by acid washing and alkaline washing.

The deprotection reaction in step 4 is generally performed in an organic solvent under acidic conditions.

In some specific embodiments, the step 4 comprises: adding the intermediate 1D and an acidic reagent to an organic solvent, then fully reacting the mixture while maintaining the temperature at 25°C ± 5°C, then dropwise adding a base while controlling the material temperature of the reaction liquid below 25°C, cooling the resulting mixture to 10°C ± 5°C for crystallization, and performing filtration and drying to obtain the compound (I); optionally, after filtration, washing the filter cake with purified water, then adding the filter cake to ethanol, stirring the mixture at 20°C ± 5°C, and performing filtration and drying to obtain the compound (I) or the salt thereof.

The advantage of using the above-mentioned conditions in the reaction of step 4 lies in that the post-treatment is simple: once the deprotection is complete, the product is directly precipitated by adding a base and obtained by filtration.

In some specific embodiments, the acidic reagent in the step 4 is selected from one or more of hydrochloric acid, sulphuric acid, phosphoric acid, formic acid, trifluoroacetic acid, benzenesulphonic acid, methanesulphonic acid and p-toluenesulphonic acid monohydrate, the organic solvent is selected from one or more of acetonitrile, acetonitrile, methanol, ethanol, isopropanol, isopropyl acetate, ethyl acetate, acetone, methyl isobutyl ketone, tetrahydrofuran, 2-methyl tetrahydrofuran and methyl tert-butyl ether, and the base is selected from one or more of dilute aqueous ammonia, LiOH, NaOH, KOH, K₃PO₄, K₂CO₃, KHCO₃, Cs₂CO₃, Na₂CO₃ and NaHCO₃.

In some specific embodiments, the material ratio range in step 4 is: 1D : acid = 1.0 : 1.0-6.0, preferably 1D : acid = 1.0 : 2.0-4.0, and the base is added dropwise in the following amount: 1D : base = 1.0 : 2.0-10.0, preferably 1D : base = 1.0 : 3.0-6.0.

In some specific embodiments, step 4 further comprises: a refining step: subjecting the obtained compound (I) to recrystallization, wherein the recrystallization reagent that can be used includes, but is not limited to acetonitrile, methanol, ethanol, isopropanol, isopropyl acetate, ethyl acetate, acetone, methyl isobutyl ketone, tetrahydrofuran, 2-methyl tetrahydrofuran, n-heptane, methyl tert-butyl ether, dimethylsulphoxide, N-methylpyrrolidone and a mixed solvent thereof.

In some specific embodiments, the refining step in step 4 comprises: adding the obtained compound (I) to a mixed solution of anhydrous ethanol-water, acetonitrile-water, acetonitrile-methanol, acetonitrile-isopropanol, acetonitrile-tert-butanol, acetonitrile-n-butanol, or acetonitrile-anhydrous ethanol, heating the mixture to 75°C ± 5°C, stirring same to dissolution and clarification, optionally performing hot filtration, cooling the filtrate under stirring for crystallization, and performing filtration and drying to obtain the refined compound (I).

In some specific embodiments, the material to liquid ratio of the crude product to the solvent (by volume) in the refining step is in the range of 1.0 : 10.0-40.0, preferably 1.0 : 12.0-20.0.

In some specific embodiments, cooling the filtrate under stirring for crystallization in step 4 is performed in two steps: first cooling the filtrate to 35°C ± 5°C and performing crystallization while maintaining the temperature constant for 20 min to 1.5 hours; and then cooling the filtrate to 5°C ± 5°C and performing crystallization while maintaining the temperature constant for 1-3 hours. The advantages of step-wise crystallization lie in excellent impurity removal and high yield.

The phrases "fully reacting" and "a complete reaction" of the present invention refer to a reaction state in which the content of the main raw material is ≤ 1.0% by sampling for HPLC monitoring.

The salt of the present invention refers to a salt formed by an acidic compound and an organic base or an inorganic base, or a salt formed by a basic compound and an organic acid or an inorganic acid, for example, triethanolamine salt, diethanolamine salt, monoethanolamine salt, carbonate, bicarbonate, hydrobromide, hydrochloride, sulphate, malate, fumarate, tartrate, oxalate, citrate, benzene sulphonate, p-toluenesulphonate, etc.

The preparation of salts is well known in the art. For example, a compound is first reacted with an acid or base in a solvent, followed by separation by precipitation, filtration, etc. to obtain the corresponding salt. Likewise, it is also well known in the art to use a salt as a substitute for a free acid or free base in chemical reactions. When the present invention describes a compound that undergoes a type of reaction to prepare another substance, it includes the circumstances where a salt form thereof is used to participate in the reaction and the product is obtained in the form of a salt. If the product is a salt, the salt can be dissolved in a solvent and subjected to a neutralization reaction by adding the corresponding acid or base to release a compound, and then the free compound can be obtained by conventional post-treatment procedures.

In the preparation method of the present invention, the entire process route is very simple to operate, and each intermediate synthesis step has a high yield, which greatly improves the overall yield and thus reduces the production cost. The intermediate products in the entire synthesis process can be crystallized and purified, have a high purity and yield, and are suitable for large-scale industrial production.

Unless otherwise specified, the operations of the present invention are carried out in accordance with conventional operations in the art. References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopaedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

### Detailed Description of Embodiments

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

### Detection method

The structure of the compound is determined by nuclear magnetic resonance (NMR) or (and) mass spectrometry (MS). The NMR shift (δ) is given in the unit of 10-6 (ppm). NMR is determined with (Bruker Avance III 400 and Bruker Avance 300) NMR instrument, and the solvent for determination is deuterated dimethyl sulphoxide (DMSO-d6), deuterated chloroform (CDCl₃), deuterated methanol (CD₃OD), and the internal standard is tetramethylsilane (TMS);
MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 µM).

### Preparation of compounds

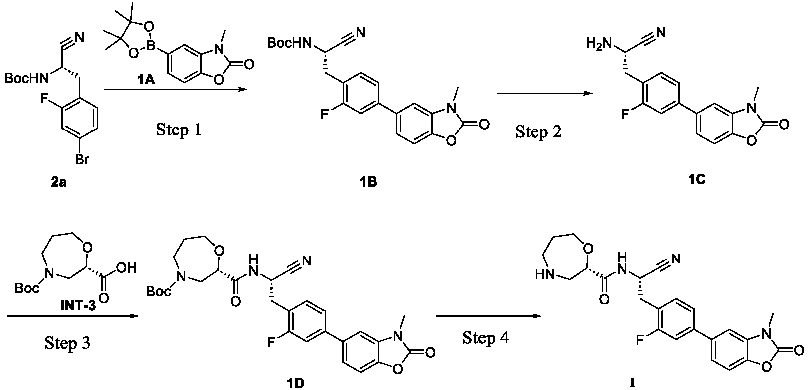

In the present invention, the target compound is prepared in four steps: coupling reaction between compound 1A and compound 2a, followed by deprotection, amidation and deprotection reactions. The method has a short route, mild conditions, simple operation, convenient post-treatment, and high yield and purity, and is suitable for industrial scale production. The target compound I has good activity, high bioavailability, low toxicity and side effects, and has druggable potential.

### Example 1

### Tert-butyl(S)-(1-cyano-2-(2-fluoro-4-(3-methyl-2-oxo-2,3-dihydrobenzo[d] oxazol-5-yl)phenyl)ethyl)carbamate (1B)

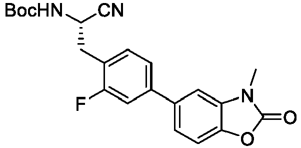

### Method I:

To a 50-L reaction kettle, 1,4-dioxane (10.005 kg), compound 1A (1.600 kg, with reference to the method in patent WO 2015110826 A1) and compound 2a (2.000 kg, with reference to the method in patent WO 2016139355 A1) were added under stirring, followed by aqueous potassium carbonate solution (6.605 kg, which was prepared by dissolving potassium carbonate (1.600 kg) in purified water (5.005 kg)). After the addition, the mixture was subjected to nitrogen replacement three times. [1,1'-bis(diphenylphosphino)ferrocene]palladium dichloride dichloromethane complex (100.0 g) was added, and the mixture was subjected to nitrogen replacement once. Under nitrogen protection, the reaction liquid was warmed to 80°C ± 5°C and reacted for about 2 hours. Then, a sample was collected and monitored with HPLC, and when the target value for in-process control, i.e., the content of compound 2a was ≤ 1.0%, the reaction was terminated.

To the reaction liquid was added purified water (5.000 kg), and the mixture was cooled to 10°C ± 5°C. Purified water (10.005 kg) was added, and the resulting mixture was stirred and crystallized for about 1 hour at 10°C ± 5°C and filtered. The filter cake was washed twice with purified water (2.500 kg × 2) and collected. Anhydrous ethanol (12.605 kg) and the filter cake were added to a 50-L reaction kettle, the mixture was stirred at 20°C ± 5°C for about 0.5 hours and filtered, and the filter cake was washed twice with anhydrous ethanol (1.000 kg × 2) and collected.

Drying: the filter cake was dried at 55°C ± 5°C under vacuum of ≤ -0.07 MPa for about 16 hours and collected to obtain intermediate 1B (2.143 kg, yield: 89.4%, HPLC: 95.91%).

1H NMR (400 MHz, DMSO) δ 7.90 (s, 1H), 7.72 - 7.30 (m, 6H), 4.72 (s, 1H), 3.41 (d, 3H), 3.09-3.21(m, 2H), 1.37 (s, 9H).

LCMS m/z =356.1[M-56+H]⁺.

### Method II:

To a 1-L reaction flask, acetonitrile (100 g), compound 1A (20.15 g) and compound 2a (16.02 g) were added under stirring, followed by aqueous potassium carbonate solution (16.57 g, which was prepared by dissolving potassium carbonate (16.53 g) in purified water (40 g)). After the addition, the mixture was subjected to nitrogen replacement three times. [1,1'-bis(diphenylphosphino)ferrocene]palladium dichloride dichloromethane complex (0.83 g) was added. Under nitrogen protection, the reaction liquid was warmed to 80°C ± 5°C and reacted for about 2 hours and then the reaction was terminated.

The reaction liquid was cooled to 50°C ± 5°C, and anhydrous ethanol (80 g) was added for crystallization for about 1 hour. The mixture was filtered and the filter cake was washed with anhydrous ethanol (20 g) and collected.

Drying: the filter cake was dried at 55°C ± 5°C under vacuum of ≤ -0.07 MPa for about 16 hours and collected to obtain intermediate 1B (24.41 g, yield: 83.0%).

### Method III:

To a 20-L reaction kettle, 1,4-dioxane (5.5 kg), compound 1A (0.551 kg) and compound 2a (0.434 kg) were added under stirring, followed by aqueous potassium carbonate solution (1.744 kg, which was prepared by dissolving potassium carbonate (0.444 kg) in purified water (1.30 kg)). After the addition, the mixture was subjected to nitrogen replacement three times. [1,1'-bis(diphenylphosphino)ferrocene]palladium dichloride dichloromethane complex (208 g) was added. Under nitrogen protection, the reaction liquid was warmed to 80°C ± 5°C and reacted for about 3 hours and then the reaction was terminated.

The reaction liquid was cooled to 20°C ± 5°C, and stirred for crystallization for about 1 hour and filtered. The filter cake was washed twice with purified water (1.0 kg × 2) and collected. Anhydrous ethanol (3.2 kg) and the filter cake were added to a 20-L reaction kettle, the mixture was warmed to 70°C ± 5°C, stirred for about 1.5 hours, cooled to room temperature and filtered, and the filter cake was washed with anhydrous ethanol (1.20 kg) and collected.

Drying: the filter cake was dried at 55°C ± 5°C under vacuum of ≤ -0.07 MPa for about 18 hours and collected to obtain intermediate 1B (0.435 kg, yield: 74.0%).

### Example 2

### (S)-2-amino-3-(2-fluoro-4-(3-methyl-2-oxo-2,3-dihydrobenzo[d]oxazol-5-yl)phenyl)propanenitrile 4-methylbenzenesulphonic acid (1C 4-methylbenzenesulphonic acid)

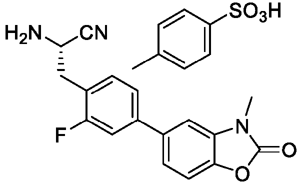

### Method I:

To a 50-L glass reaction kettle, acetonitrile (16.785 kg), intermediate 1B (2.1381 kg) and p-toluenesulphonic acid monohydrate (2.950 kg) were added under stirring. After the addition, the temperature was controlled at 25°C ± 5°C and the mixture was reacted for about 2 hours. Then, a sample was collected and monitored with HPLC, and when the target value for in-process control, i.e., the content of intermediate 1B was ≤ 1.0%, the reaction was terminated.

The reaction mixture was filtered, and the filter cake was washed with acetonitrile (1.670 kg) and collected. Acetonitrile (2.1381 kg) and filter cake were added to a reaction kettle, and the mixture was warmed to 80°C ± 5°C and stirred for 3 hours. The resulting mixture was cooled to 20°C ± 5°C and stirred for 1 hour. The reaction mixture was filtered, and the filter cake was washed with acetonitrile (1.670 kg) and collected.

Drying: the filter cake was dried at 55°C ± 5°C under vacuum of ≤ -0.07 MPa for about 16 hours and collected to obtain intermediate 1C 4-methylbenzenesulphonic acid (2.141 kg, yield: 85.5%, HPLC: 99.43%).

1H NMR (400 MHz, DMSO) δ 9.04 (s, 3H), 7.70 - 7.37 (m, 8H), 7.13 (d, 2H), 4.90 (dd, 1H), 3.41 (s, 3H), 3.29 (t, 2H), 2.29 (s, 3H).

LCMS m/z =312.2 [M-172+H]⁺.

### Method II:

To a 1-L reaction flask, acetonitrile (400 g), intermediate 1B (50.30 g) and p-toluenesulphonic acid monohydrate (69.30 g) were added under stirring. After the addition, the temperature was controlled at 50°C ± 5°C and the mixture was reacted for about 1 hours. Then, a sample was collected and monitored with HPLC, and when the in-process control content of intermediate 1B was ≤ 1.0%, the reaction was terminated. The reaction mixture was filtered, and the filter cake was washed with acetonitrile (10 g) and collected.

Drying: the filter cake was dried at 55°C ± 5°C under vacuum of ≤ -0.07 MPa for about 24 hours and collected to obtain intermediate 1C 4-methylbenzenesulphonic acid (55.70 g, yield: 95.0%).

### Method III:

To a 0.5-L reaction flask, acetonitrile (160 g), intermediate 1B (20.3 g) and concentrated hydrochloric acid (17.8 g) were added under stirring. After the addition, the temperature was controlled at 25°C ± 5°C and the mixture was reacted for about 2 hours. Then, a sample was collected and monitored with HPLC, and when the in-process control content of intermediate 1B was ≤ 1.0%, the reaction was terminated. The reaction mixture was filtered, and the filter cake was washed with acetonitrile (10 g) and collected.

Drying: the filter cake was dried at 55°C ± 5°C under vacuum of ≤ -0.07 MPa for about 18 hours and collected to obtain the hydrochloride of intermediate 1C (14.4 g, yield: 85.0%, hygroscopic).

### Example 3

### Tert-butyl(S)-2-(((S)-1-cyano-2-(2-fluoro-4-(3-methyl-2-oxo-2,3-dihydrobenzo [d]oxazol-5-yl)phenyl)ethyl)carbamoyl)-1,4-oxazepane-4-carboxylate (1D)

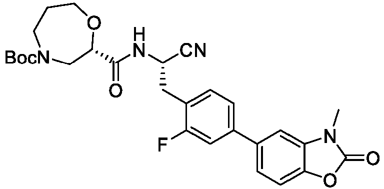

### Method I:

To a 50-L reaction kettle, ethyl acetate (19.060 kg), intermediate 1C (2.1413 kg) and INT-3 (1.1300 kg, which was purchased from Pharmablock Sciences (Nanjing), Inc.) were added under stirring, followed by N,N-diisopropylethylamine (1.725 kg). After the addition, under nitrogen protection, the reaction liquid was cooled to 5°C ± 5°C, propylphosphonic anhydride (4.240 kg) was added dropwise while the temperature was controlled at 10°C ± 5°C. After the addition, the temperature was maintained at 25°C ± 5°C and the mixture was reacted for about 2 hours. Then, a sample was collected and monitored with HPLC, and when the target value for in-process control, i.e., the content of intermediate 1C was ≤ 1.0%, the reaction was terminated.

The reaction liquid was washed sequentially with sodium bicarbonate solution (which was prepared by dissolving 1.070 kg of sodium bicarbonate in 20.350 kg of water), citric acid solution (which was prepared by dissolving 2.150 kg of citric acid monohydrate in 19.275 kg of water) and sodium chloride (which was prepared by dissolving 4.300 kg of sodium chloride in 17.135 kg of water). To the organic phase was added medicinal charcoal (0.210 kg), and the mixture was stirred for about 0.5 hours. The resulting mixture was filtered through a pad of celite (0.540 kg), and the filter cake was washed with ethyl acetate (1.905 kg). The organic phase was dried for about 0.5 hours by adding anhydrous sodium sulphate (1.070 kg). The mixture was filtered, the filter cake was washed with ethyl acetate (1.905 kg), and the filtrates were combined.
the resulting filtrate was concentrated under reduced pressure at 50°C ± 5°C until no obvious fraction flew out, to obtain intermediate 1D (2.385 kg, overweight, and the amount was calculated based on a 100% yield). The intermediate was directly used for the next reaction (HPLC: 99.09%).

1H NMR (400 MHz, CDCl3) δ 7.54 - 7.01 (m, 7H), 5.18 (s, 1H), 4.25 - 3.94 (m, 3H), 3.54 (dd, 2H), 3.46 (s, 3H), 3.39 - 3.04 (m, 4H), 1.99 (d, 2H), 1.54 - 1.39 (m, 9H).

LCMS m/z =483.2 [M-56+1]⁺.

### Method II:

To a 2-L reaction flask, dichloromethane (1.3 kg), intermediate 1C (120.03 g) and INT-3 (108.76 g) were added under stirring, followed by N,N-diisopropylethylamine (149.55 g). After the addition, the reaction liquid was cooled to 15°C ± 5°C, and HATU (190.47 g) was added in batches. After the addition, the mixture was reacted for about 16 hours while the temperature was maintained at 25°C ± 5°C and then the reaction was terminated.

The reaction liquid was washed sequentially with sodium bicarbonate solution, citric acid solution, and sodium chloride. The organic phase was dried for about 0.5 hours by adding anhydrous sodium sulphate (1.0 kg). The mixture was filtered, the filter cake was washed with dichloromethane (1.905 kg), and the filtrates were combined.
the resulting filtrate was concentrated under reduced pressure at 50°C ± 5°C until no obvious fraction flew out, to obtain intermediate 1D (221.30 g, overweight, and the amount was calculated based on a 100% yield). The intermediate was directly used for the next reaction.

### Method III:

To a 1-L reaction flask, ethyl acetate (800 g), intermediate 1C (100.0 g) and INT-3 (53.26 g) were added under stirring, followed by N,N-diisopropylethylamine (80.11 g). After the addition, under nitrogen protection, the reaction liquid was cooled to 5°C ± 5°C, propylphosphonic anhydride (197.66 g) was added dropwise while the temperature was controlled at 10°C ± 5°C. After the addition, the temperature was maintained at 25°C ± 5°C and the mixture was reacted for about 2 hours. Then, a sample was collected and monitored with HPLC, and when the target value for in-process control, i.e., the content of intermediate 1C was ≤ 1.0%, the reaction was terminated.

The reaction liquid was washed sequentially with sodium bicarbonate solution, citric acid solution, and sodium chloride. The organic phase was dried for about 0.5 hours by adding anhydrous sodium sulphate (200 g). The mixture was filtered, the filter cake was washed with ethyl acetate (50 g), and the filtrates were combined.
the resulting filtrate was concentrated under reduced pressure at 50°C ± 5°C until no obvious fraction flew out, to obtain intermediate 1D (115.08 g, overweight, and the amount was calculated based on a 100% yield). The intermediate was directly used for the next reaction.

### Example 4

### (S)-N-((S)-1-cyano-2-(2-fluoro-4-(3-methyl-2-oxo-2,3-dihydrobenzo[d]oxazol-5-yl)phenyl)ethyl)-1,4-oxazepane-2-carboxamide (compound I)

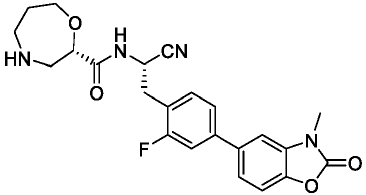

### Method I:

To a 50-L double-layer glass reaction kettle containing the concentrate of intermediate 1D (method I of Example 3), acetonitrile (9.305 kg) and p-toluenesulphonic acid monohydrate (2.530 kg) were added under stirring. After the addition, the temperature was maintained at 25°C ± 5°C and the mixture was reacted for about 2 hours. Then, a sample was collected and monitored with HPLC, and when the target value for in-process control, i.e., the content of intermediate 1D was ≤ 1.0%, the reaction was terminated.

The reaction liquid was cooled to 10°C ± 5°C, and dilute aqueous ammonia (a mixture of 1.075 kg of aqueous ammonia and 36.000 kg of purified water) was added dropwise while the material temperature was controlled below 25°C. After the addition, the mixture was cooled to 10°C ± 5°C to allow crystallization for 2 hours. The resulting mixture was filtered, and the filter cake was washed with purified water (11.930 kg). The filter cake and ethanol (14.890 kg) were added to a 50-L double-layer glass reaction kettle and the mixture was stirred at 20°C ± 5°C for 0.5 hours and filtered. The filter cake was washed with ethanol (1.860 kg) and collected. The filter cake was dried at 55°C ± 5°C under vacuum (vacuum degree ≤ -0.07 MPa) for about 13 hours and collected to obtain crude compound I (1.6627 kg, yield: 85.6%).

Refining: to a 100-L reaction kettle, acetonitrile (9.100 kg), anhydrous ethanol (9.220 kg) and crude compound I (1.6627 kg) were added under stirring, and the mixture was heated to an internal temperature of 75°C ± 5°C, stirred to dissolution and clarification, and filtered while hot. The filtrate was transferred into a 100-L reaction kettle (if a product was precipitated out from the filtrate, the filtrate should be heated until the solution was clear) and cooled to 35°C ± 5°C under stirring. The temperature was maintained constant until a solid was precipitated out and then the mixture was stirred for about 0.5 hours while the temperature was maintained constant. The resulting mixture was then cooled to 5°C ± 5°C, and the temperature was maintained constant to allow crystallization for 2 hours. The mixture was filtered, and the filter cake was washed with ethanol (1.300 kg) and collected.

Drying: the filter cake was dried at 55°C ± 5°C under vacuum of ≤ -0.07 MPa for about 25 hours to obtain compound I (1.4060 kg, yield: 84.6%, total yield: 55.0% (calculated based on compound 2a), HPLC: 99.93%).

1H NMR (400 MHz, DMSO) δ 8.69 (d, 1H), 7.64 (d, 1H), 7.61 - 7.51 (m, 2H), 7.46 (t, 2H), 7.39 (d, 1H), 5.06 (q, 1H), 4.01 (dd, 1H), 3.87 (ddd, 1H), 3.73 (ddd, 1H), 3.40 (s, 3H), 3.34 - 3.28 (m, 1H), 3.20 (dd, 1H), 3.06 (dd, 1H), 2.78 (ddd, 1H), 2.69 - 2.54 (m, 2H), 2.21 (s, 1H), 1.84 - 1.63 (m, 2H).

LCMS m/z =439.2 [M+1]⁺.

### Method II:

To a 100-L double-layer glass reaction kettle, acetonitrile (19.26 kg), p-toluenesulphonic acid monohydrate (3.712 kg) and intermediate 1D (3.500 kg) were added under stirring. After the addition, the temperature was maintained at 25°C ± 5°C and the mixture was reacted for about 2 hours. Then, a sample was collected and monitored with HPLC, and when the target value for in-process control, i.e., the content of intermediate 1D was ≤ 1.0%, the reaction was terminated.

The reaction liquid was concentrated to a reaction volume of about 20 L and cooled to 10°C ± 5°C, and dilute aqueous ammonia (a mixture of 4.0 kg of aqueous ammonia and 56.00 kg of purified water) was added. After the addition, the mixture was cooled to 10°C ± 5°C to allow crystallization for 0.5 hours. The mixture was filtered, and the filter cake was washed with purified water (5 kg) and collected. The filter cake was dried at 55°C ± 5°C under vacuum (vacuum degree ≤ -0.07 MPa) for about 72 hours and collected to obtain crude compound I (2.552 kg).

Refining: to a 100-L reaction kettle, isopropanol (19.63 kg) and crude compound I (1.6627 kg) were added under stirring, the mixture was heated to an internal temperature of 75°C ± 5°C, stirred for about 2 hours and then cooled to 25°C ± 5°C, and the temperature was maintained constant to allow crystallization for about 16 hours. The mixture was filtered, and the filter cake was washed with isopropanol (1.300 kg) and collected.

Drying: the filter cake was dried at 55°C ± 5°C under vacuum of ≤ -0.07 MPa for about 24 hours to obtain compound I (2.344.2 kg, yield: 82.3%).

### Biological test

### 1. In vitro DPP1 enzyme activity assay

Recombinant human DPP1 enzyme (R&D Systems, Cat. No. 1071-CY) at a final concentration of 100 µg/mL and recombinant human cathepsin L (R&D Systems, Cat. No. 952-CY) at a final concentration of 20 µg/mL were mixed and incubated at room temperature for 1 hour to activate the DPP1 enzyme. The activated DPP1 enzyme was diluted 100-fold, and 5 µL of compounds at different concentrations and 5 µL of the diluted DPP1 enzyme were added to a 384-well plate and incubated at room temperature for 30 minutes. After 10 µL of the substrate Gly-Arg-AMC (bachem, Cat. No. I-1215) at a concentration of 20 µM was added, incubation was continued at room temperature for 60 minutes, and the fluorescence intensity was detected with a microplate reader (excitation = 380 nm and emission = 460 nm). The IC50 values were calculated by using the DosResp function in an Origin2019 software.

**Table 1 DPP1 inhibitory activity**

| Compound No. | IC₅₀/nM |
|---|---|
| Compound I | 1.6 |

| | |
|---|---|
| Conclusion: the compound of the present invention shows relatively high inhibitory activity against the DPP1 receptor. | |

### 2. Pharmacokinetic test in rats

1.1 Experimental animals: male SD rats, about 220 g, 6-8 weeks old, 6 rats/compound. Purchased from Chengdu Ddossy Experimental Animals Co., Ltd.
1.2 Experimental design: On the day of the experiment, 6 SD rats were randomly grouped according to their body weights. The animals were fasted with water available for 12 to 14 h one day before the administration, and were fed 4 h after the administration.

**Table 2 Administration information**

| Group | Number | Administration information | | | | | |
|---|---|---|---|---|---|---|---|
| | Male | Test compound | Administrati on dosage (mg/kg) | Administration concentration (mg/mL) | Administrati on volume (mL/kg) | Collected samples | Mode of administration |
| G1 | 3 | INS1007 | 1 | 0.2 | 5 | Plasma | Intravenously |
| G2 | 3 | Compound I | 1 | 0.2 | 5 | Plasma | |
| G3 | 3 | INS1007 | 3 | 0.3 | 10 | Plasma | Intragastrically |
| G4 | 3 | Compound I | 3 | 0.3 | 10 | Plasma | |

Vehicle for intravenous administration: 5% DMA + 5% Solutol + 90% Saline; vehicle for intragastric administration: 0.5% MC; the control compound INS1007, i.e., compound 2 in patent WO 2015110826 A1, was prepared according to the method in the patent.

Before and after the administration, 0.1 ml of blood was taken from the orbit of the rats under isoflurane anaesthesia, and placed in an EDTAK2 centrifuge tube. Centrifugation was carried out at 5000 rpm at 4°C for 10 min and the plasma was collected. Blood sampling time points for the intravenous administration group: 0, 5 min, 15 min, 30 min, 1 h, 2 h, 4 h, 6 h, 8 h, and 24 h; and blood sampling time points for the intragastric administration group: 0, 5 min, 15 min, 30 min, 1 h, 2 h, 4 h, 6 h, 8 h, and 24 h. Before analysis and detection, all samples were stored at - 80°C.

**Table 3 Pharmacokinetic parameters of test compounds in plasma of rats**

| Test compounds | Mode of administration | CL (mL/min/kg) | Vdss (L/kg) | AUC0-t (hr*ng/mL) | F (%) |
|---|---|---|---|---|---|
| INS1007 | Intravenously (1 mg/kg) | 2.08 | 0.738 | 8396 | - |
| Compound 1 | | 1.66 | 0.753 | 9503 | - |
| INS1007 | Intragastrically (3 mg/kg) | - | - | 22201 | 88.1 |
| Compound 1 | | - | - | 31159 | >100 |

| | | | | | |
|---|---|---|---|---|---|
| Conclusion: the compound of the present invention has relatively good bioavailability and pharmacokinetic characteristics. | | | | | |

### 3. Toxicity test in rats following 14-day repeated oral administration

According to their body weights, the SD rats were randomly divided into the following groups: vehicle control group (0.5% MC), INS1007 (30, 100, and 300 mg/kg) groups, and compound I (30, 100, and 300 mg/kg) groups. For the administration groups, 16 rats were included in each group, and for the vehicle control group, 10 rats were included, with an equal number of male and female rats in each of the groups. The rats were orally gavaged with the drug or vehicle at the corresponding concentrations every day for 14 consecutive days, with a recovery period of 7 days. During the administration period, general symptoms were observed, and body weight and food intake were measured for each group. At the end of the administration period and at the end of the recovery period, haematology tests, serum biochemistry test and gross anatomy were performed on the rats in each group separately.

Conclusion: the compound of the present invention is less toxic than INS 1007 and thus is safer at the same dose.

## Claims

1. A preparation method of a compound represented by formula (I), 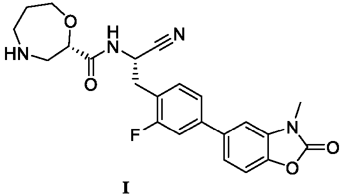
**characterised in that** the preparation method comprises: step 1: subjecting compound 2a and compound 1A to a coupling reaction to obtain intermediate 1B; 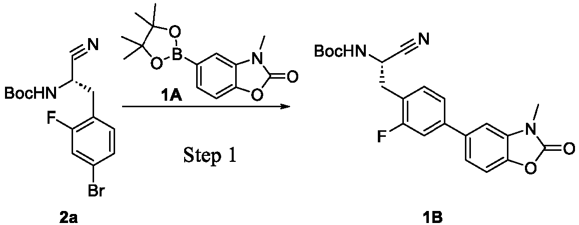
and step 2: adding the intermediate 1B and an acid to an organic solvent, fully reacting the mixture while controlling the temperature at 20°C-55°C, and then filtering same to obtain intermediate 1C or a salt thereof; optionally, adding the filter cake to an organic solvent, warming the mixture to 80°C ± 5°C and stirring same for 1 to 5 hours, cooling same to 20°C ± 5°C, stirring same and performing filtration and drying to obtain the intermediate 1C or the salt thereof,
wherein the acid is selected from one or more of sulphuric acid, phosphoric acid, formic acid, trifluoroacetic acid, benzenesulphonic acid, methanesulphonic acid and p-toluenesulphonic acid monohydrate; and the organic solvent is selected from one or more of methanol, ethanol, isopropanol, acetonitrile, isopropyl acetate, ethyl acetate, toluene, acetone, methyl isobutyl ketone, tetrahydrofuran, 2-methyl tetrahydrofuran, methyl tert-butyl ether and acetonitrile;

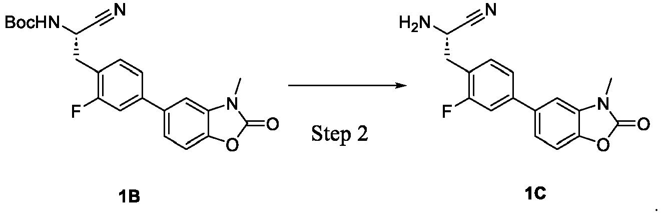

2. The preparation method according to claim 1, **characterised in that** the step 1 comprises: dissolving the compound 2a and the compound 1A in an organic solvent, adding a base, then adding a catalyst under nitrogen protection and warming the mixture to 80°C ± 5°C for a complete reaction; then cooling the reaction mixture to 5°C-55°C, stirring same for crystallization, and performing filtration and drying to obtain the intermediate 1B; optionally, after filtration, adding the filter cake to an alcohol solvent, stirring the mixture for 10 min to 2 h, and then performing filtration and drying to obtain the intermediate 1B,
wherein the organic solvent is selected from one or more of acetonitrile, toluene, dichloromethane, ethyl acetate, acetone, methanol, ethanol, isopropanol, 2-methyl tetrahydrofuran, tetrahydrofuran and 1,4-dioxane;
the base is selected from one or more of K₃PO₄, K₂HPO₄, K₂CO₃, KHCO₃, Cs₂CO₃, Na₂CO₃, NaHCO₃, KF, sodium acetate, potassium acetate, pyridine, triethylamine or N,N-diisopropylethylamine, or an aqueous solution thereof;
and the catalyst is selected from one or more of NiCl₂, NiCl₂·diglyme, Ni(COD)₂, Pd(OAc)₂, PdCl₂, Pd(PPh₃)₂Cl₂, Pd(dppf)Cl₂, Pd(PPh₃)₄, Pd₂(dba)₃, Pd(PhCN)₂Cl₂, PEPPSI-iPr, PdCl₂[P(Cy)₃]₂, and [1,1'-bis(diphenylphosphino)ferrocene]palladium dichloride dichloromethane complex.

3. The preparation method according to claim 1 or 2, **characterised in that** the preparation method further comprises:
step 3: subjecting the intermediate 1C or the salt thereof and compound INT-3 or a salt thereof to an amidation reaction to obtain intermediate 1D; 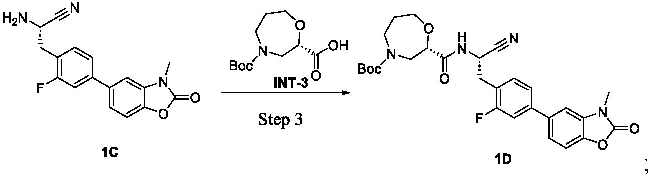 or/and
step 4: subjecting the intermediate 1D to deprotection under acidic conditions to obtain compound (I) or a salt thereof;

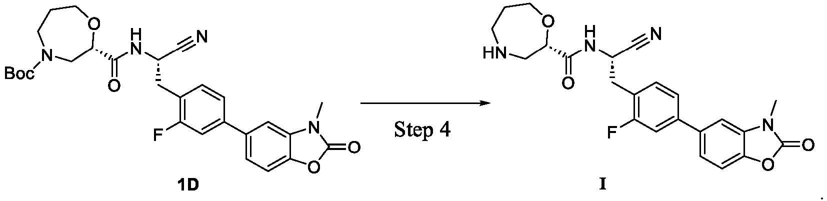

4. The preparation method according to claim 3, **characterised in that** the step 3 comprises: adding the intermediate 1C or the salt thereof and the compound INT-3 or the salt thereof to an organic solvent, adding an organic amine, then dropwise adding an acid-amine condensation reagent under nitrogen protection while controlling the temperature at 5°C-20°C, and then fully reacting the mixture while maintaining the temperature at 25°C ± 5°C; then, washing the reaction liquid with sodium chloride solution, performing liquid separation, adding activated carbon or medicinal charcoal to the organic phase, stirring and filtering the mixture, drying the organic phase by adding anhydrous sodium sulphate, and performing filtration and concentration to obtain the intermediate 1D or the salt thereof,
wherein the organic solvent is selected from one or more of tetrahydrofuran, 2-methyl tetrahydrofuran, dioxane, N,N-dimethylformamide, N,N-dimethylacetamide, N-methylpyrrolidone, dimethylsulphoxide, dichloromethane, acetone, methyl isobutyl ketone, isopropyl acetate and ethyl acetate;
the organic amine is selected from one or more of triethylamine, 2,6-dimethylpyridine, pyridine, DBU and N,N-diisopropylethylamine;
and the acid-amine condensation reagent is selected from one or more of HATU, COMU, EDCI, BOP and propylphosphonic anhydride.

5. The preparation method according to claim 4, **characterised in that** the step 3 comprises: sequentially washing the reaction liquid with an alkaline aqueous solution and an acidic aqueous solution before washing same with sodium chloride solution,
wherein the alkaline aqueous solution is selected from one or more of potassium phosphate solution, potassium carbonate solution, potassium bicarbonate solution, sodium carbonate solution and sodium bicarbonate solution;
and the acidic aqueous solution is selected from one or more of hydrochloric acid solution, sulphuric acid solution, phosphoric acid solution and citric acid solution.

6. The preparation method according to claim 3, **characterised in that** the step 4 comprises: adding the intermediate 1D and an acidic reagent to an organic solvent, then fully reacting the mixture while maintaining the temperature at 25°C ± 5°C, then dropwise adding a base while controlling the material temperature of the reaction liquid below 25°C, cooling the resulting mixture to 10°C ± 5°C for crystallization, and performing filtration and drying to obtain the compound (I); optionally, after filtration, washing the filter cake with purified water, then adding the filter cake to an alcohol reagent, stirring the mixture at 20°C ± 5°C, and performing filtration and drying to obtain the compound (I) or the salt thereof.

7. The preparation method according to claim 6, **characterised in that** the acidic reagent in the step 4 is selected from one or more of hydrochloric acid, sulphuric acid, phosphoric acid, formic acid, trifluoroacetic acid, benzenesulphonic acid, methanesulphonic acid and p-toluenesulphonic acid monohydrate, the organic solvent is selected from one or more of acetonitrile, methanol, ethanol, isopropanol, isopropyl acetate, ethyl acetate, acetone, methyl isobutyl ketone, tetrahydrofuran, 2-methyl tetrahydrofuran and methyl tert-butyl ether, and the base is selected from one or more of dilute aqueous ammonia, LiOH, NaOH, KOH, K₃PO₄, K₂CO₃, KHCO₃, Cs₂CO₃, Na₂CO₃, NaHCO₃, or an aqueous solution thereof.

8. The preparation method according to claim 6 or 7, **characterised in that** the preparation method further comprises: a refining step: adding the obtained compound (I) to a solution of anhydrous ethanol-water, acetonitrile-water, acetonitrile-anhydrous ethanol, acetonitrile-methanol, acetonitrile-isopropanol, acetonitrile-tert-butanol, or acetonitrile-n-butanol, heating the mixture to 75°C ± 5°C, stirring same to dissolution and clarification, optionally performing hot filtration, cooling the filtrate under stirring for crystallization, and performing filtration and drying to obtain the refined compound (I).

9. The preparation method according to claim 8, **characterised in that** cooling the filtrate under stirring for crystallization is performed in two steps: first cooling the filtrate to 35°C ± 5°C and performing crystallization while maintaining the temperature constant for 20 min to 1.5 hours; and then cooling the filtrate to 5°C ± 5°C and performing crystallization while maintaining the temperature constant for 1-3 hours.
